Europäisches Patentamt

European Patent Office

Office européen des brevets

(19)

(11) Veröffentlichungsnummer: **0 195 944 B1**

# (12) EUROPÄISCHE PATENTSCHRIFT

(45) Veröffentlichungstag der Patentschrift: **09.10.91**

(51) Int. Cl.⁵: **C12P 13/00**

(21) Anmeldenummer: **86102438.8**

(22) Anmeldetag: **25.02.86**

(54) **Verfahren zur kontinuierlichen Herstellung von L-Carnitin.**

(30) Priorität: **27.02.85 CH 890/85**

(43) Veröffentlichungstag der Anmeldung:
**01.10.86 Patentblatt 86/40**

(45) Bekanntmachung des Hinweises auf die
Patenterteilung:
**09.10.91 Patentblatt 91/41**

(84) Benannte Vertragsstaaten:
**AT BE CH DE FR GB IT LI LU NL SE**

(56) Entgegenhaltungen:
**DD-A- 221 905**
**GB-A- 2 048 877**
**GB-A- 2 078 742**

(73) Patentinhaber: **LONZA AG**

**Gampel/Wallis(CH)**

(72) Erfinder: **Kulla, Hans, Dr.**
**Wildi**
**Visp Kanto Wallis(CH)**
Erfinder: **Lehky, Pavel, Dr.**
**Dammweg 13**
**Naters Kanton Wallis(CH)**
Erfinder: **Squaratti, Armand**
**Potence F.**
**Siders Kanton Wallis(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

**Beschreibung**

Die Erfindung betrifft ein neues kontinuierliches Verfahren zur Herstellung von L-Carnitin auf mikrobiologischem Weg.

Es ist bekannt, L-Carnitin aus $\gamma$-Butyrobetain herzustellen. Das $\gamma$-Butyrobetain Wird in Gegenwart von Natrium-2-oxoglutarat, einem reduzierenden Agens, einer Eisenionenquelle und einem Luftsauerstoff als Hydroxylgruppendonor mit einem Hydroxylase-Enzym, freigesetzt aus Sporen von Neurospora crassa, in Berührung gebracht (US-PS 4 371 618). Dieses Verfahren weist den Nachteil auf, eine Vielzahl von Cofaktoren zu benötigen, die extern geführt werden müssen.

So werden in der Reaktion stöchiometrische Mengen 2-Oxoglutarat oxidativ zu Succinat decarboxyliert. $Fe^{2+}$ wird als $O_2$-Aktivator benötigt, Ascorbat, um das Eisen-Ion in der reduzierten Form zu halten, und Katalase, um das in Spuren entstehende, schädliche $H_2O_2$ zu zerstören.

Lindstedt et al, (Biochemistry 6, 1262-1270 (1967) "The Formation and Degradation of Carnitin in Pseudomonas") isolierten einen Mikroorganisumus der Gattung Pseudomonas, der mit $\gamma$-Butyrobetain als C- und N-Quelle wächst. Die erste Reaktion des Abbauweges war die Hydroxylierung des $\gamma$-Butyrobetains zu L-Carnitin, wobei das intermediär entstehende L-Carnitin vollständig zu $CO_2$, $H_2O$ und $NH_3$ weiterkatabolisiert wurde.

Auch diese, aus Bakterien gewonnene Hydroxylase hätte, wenn sie für die L-Carnitin-Produktion eingesetzt werden würde, die geschilderten nachteiligen Cofaktor-Bedürfnisse [Lindstedt et al, Biochemistry 16, 2181- 2188 (1977)] "Purification and Properties of $\gamma$-Butyrobetaine Hydroxylase from Pseudomonas sp. $\overline{AK}$ 1").

Die Aufgabe der vorliegenden Erfindung ist es die Nachteile der bekannten Verfahren zu vermeiden und ein Verfahren zu finden, dass es gestattet in einem kontinuierlichem System, enantioselektiv und auf mikrobiologischem Weg L-Carnitin aus Crotonobetain und oder $\gamma$-Butyrobetain herzustellen.

Im Unterschied zu den im Stand der Technik bekannten Systemen, verwenden unsere Mikroorganismen das $H_2O$ und nicht das $O_2$ als Hydroxylgruppendonor, wie durch eigene Untersuchungen unter Verwendung von $H_2^{18}O$ und $^{18}O_2$ festgestellt werden konnte.

Erfindungsgemäss wird die Aufgabe dadurch gelöst, dass man in einem Bioreaktor den Mikroorganismus 1331b (DSM Nr. 3225) oder seine Mutanten mit Cronobetain und/oder $\gamma$-Butyrobetain in Gegenwart eines Wachstumssubstrats kultiviert und die Kulturflüssigkeit ausserhalb des Bioreaktors in einen Kreislauf führt, in welchem eine Zellabtrennung durchgeführt wird, wobei dem Bioreaktor eine gleich grosse Menge zellfreier Lösung entzogen wird wie als Substrat dem Bioreaktor zugeführt wird und dadurch, dass man schliesslich aus der zellfreien Lösung das L-Carnitin abtrennt.

Es kann mit dieser vorteilhaften Verfahrensweise, durch die Biomasseretention, eine höhere Produktivität sowie eine stark verbesserte Langzeitstabiliät des kontinuierlichen Verfahrens erreicht werden.

Der bevorzugte Mikoorganisumus HK 1331b, hinterlegt am 8.2.1985 bei der Deutschen Sammlung von Mikroorganismen (DSM), Gesellschaft für Biotechnologische Forschung mbH, Griesebachstr. 8, D-4300 Göttingen, unter der Nr. DSM 3225 ist besonders befähigt im erfindungsgemässen kontinuierlichen System aus Crotonobetain und / oder $\gamma$-Butyrobetain L-Carnitin zu produzieren und letzteres nicht zu katabolisieren.

Dieser Mikroorganismus ist erhältlich durch folgende Selektionsmethoden:

a) Mikroorganismen, die mit Betain, $\gamma$-Butyrobetain, Crotonobetain und L-Carnitin als C- und N-Quelle wachsen, werden auf übliche Weise mutiert.

b) Aus der durch Züchtung erhaltenen Kultur von mutierten Mikroorganismen werden jene selektioniert, die stabil sind, L-Carnitin nicht katabolisieren und nicht auf L-Carnitin, Crotonobetain, $\gamma$-Butyrobetain wachsen, wohl aber mit Betain.

c) Aus letzterer Kultur wurde ein solcher Stamm selektioniert, der stabil ist,L-Carnitin nicht katabolisiert und nicht auf L-Carnitin, Crotonobetain, $\gamma$-Butyrobetain wächst, und gutes Wachstum in einem Medium zeigt, das sowohl L-Glutamat wie auch $\gamma$-Butyrobetain oder Crotonobetain enthält.

Vorzugsweise werden folgend auf Selektionsschritt b) jene Mikroorganismen ausgewählt, die L-Carnitin ausscheiden und nicht auf L-Carnitin, Crotonobetain, $\gamma$-Butyrobetain wachsen, wohl aber mit Betain.

Zweckmässig werden die mutierten Mikroorganismen in einem Betain-Medium weiterkultiviert und diese weiterkultivierten Mikroorganismen zur Durchführung des Selektionsschrittes b) vorzugsweise in einem L-Carnitin-Medium weitergezüchtet. Die Züchtung von mit Betain, $\gamma$-Butyrobetain, Crotonobetain und L-Carnitin als C- und N-Quelle wachsenden Stämme wird zweckmässig so durchgeführt, dass man aus Bakteriengemischen durch Beimpfung von Crotonobetain-Nährlösungen Mischkulturen erzeugt und aus denen unter Zuhilfenahme traditioneller mikrobiologischer Techniken dann Reinkulturen von Crotonobetain abbauenden Mikroorganismen anlegt.

Die Mutation einer solchen Kultur, die mit Betain, $\gamma$-Butyrobetain, Crotonobetain und L-Carnitin als C- und N-Quelle wächst, kann nach bekannten Methoden durchgeführt werden [J.H. Miller, Experiments in Molecular Genetics, Cold Spring Harbor Laboratory, 1972].

Zweckmässig anwendbare Methoden zur Herstellung stabiler Mutanten sind die Frame-Shift-Methode, Deletionsmethode oder die Transposon-Insertionsmethode.

Die so mutierten Mikroorganismen werden dann nach der Weiterkultivierung in einem Betain-Medium und der Ueberführung in ein L-Carnitin-Medium dem Selektionsschritt b) unterzogen, wo mittels bekannten "counter-selektionierenden Agenzien" [P.Gerhardt et al (eds.), Manual of Methods for General Bacteriology, Am.Soc. for Microbiology, 1981] jene Mikroorganismen selektioniert werden, die stabil L-Carnitin nicht katabolisieren und nicht auf L-Carnitin, Crotonobetain, $\gamma$-Butyrobetain wachsen, wohl aber mit Betain.

Ausgehend von diesen L-Carnitin produzierenden Stämmen wurden entsprechend Schritt c spontane, gut wachsende Kolonien von der Oberfläche eines mit Agar verfestigten Nährmediums, das L-Glutamat und Butyrobetain (wahlweise auch Crotonobetain oder L-Carnitin) enthält, isoliert.

Diese Stämme wachsen schlecht mit Betain. Sie sind somit ideal im erfindungsgemässen kontinuierlichen Verfahren mit Biomasseretention einsetzbar, da sich in einem Betain plus Butyrobetain (wahlweise auch Crotonobetain) Medium ein produktives Gleichgewicht, ohne Biomassezuwachs, einstellt.

Das in der Anlaufphase erwünschte schnelle Anwachsen kann durch Zugabe von L-Glutamat erreicht werden.

Dieser bevorzugte Stamm ist der HK 1331b (DSM Nr. 3225) sowie dessen Deszendenten und Mutanten.

## Wissenschaftliche Beschreibung des Stammes HK 1331 b

| | | | |
|---|---|---|---|
| Zellform | Stäbchen z.T.pleomorph | Phenylalanindeaminase | − |
| Länge µm | 1-2 | Ornithindecarboxylase | − |
| Breite µm | 0,5-0,8 | | |
| Beweglichkeit | + | $H_2S$ | − |
| Geisseln | peritrich | Voges-Proskauer | − |
| Gram-Reaktion | − | Indol | − |
| Sporen | − | Nitrit aus Nitrat | + |
| Bildung von Poly-β-hydro-xybutyrat | − | Denitrifikation | + |
| | | Levanbildung | − |
| Oxidase | + | Lecithinase | − |
| Catalase | + | Urease | + |
| Wachstum | | Abbau von | |
| anaerob | − | Stärke | − |
| 37°C | + | Gelatine | − |
| 41°C | − | Casein | − |
| pH 5,6 | − | Tyrosin | − |
| Mac-Conkey-Agar | + | Tween 80 | − |
| SS-Agar | − | DNA | + |
| Cetrimid-Agar | − | Aesculin | + |
| Pigmentbildung | | Substratverwertung | |
| nicht diffundierend | − | Acetat | − |
| diffundierend | − | Citrat | − |
| fluoreszierend | − | Malonat | − |
| Säurebildung (OF-Test) aus | | Glycin | − |
| Glucose aerob | − | Norleucin | − |
| anaerob | − | Xylose | + |
| Fructose aerob | − | Fructose | + |
| ASS Glucose | + | Glucose | + |
| Xylose | + | Autotrophes Wachstum mit $H_2$ | − |
| Trehalose | + | | |
| Aethanol | − | 3-Ketolactose | − |

| | | | |
|---|---|---|---|
| Gasbildung aus Glucose | – | Wachstum | |
| ONPG | + | Betain | + |
| Arginindihydrolase | – | L-Carnitin | – |
| Lysindecarboxylase | – | γ-Butyrobetain | – |
| | | Crotonobetain | – |
| | | L-Glutamat und Crotonbetain | + |
| | | L-Glutamat und Butyrobetain | + |
| | | L-Glutamat und L-Carnitin | + |

Das kontinuierliche Verfahren zur Herstellung von L-Carnitin wird zweckmässig derart ausgeführt, dass man eine Vorkultur des Stammes HK 1331b in einem sterilisierten, vorzugsweise vitaminhaltigen Mineralmedium [Kulla et al, Arch. Microbiol. 135, 1 (1983)] bie 20 bis 40°C, vorzugsweise bei 30°C, bei einem zweckmässigen pH-Wert von 6 bis 8, vorzugsweise 7, während 20 bis 50 Stunden, vorteilhaft während 30 bis 40 Stunden, kultiviert. Diese Vorkultur enthält zweckmässig 0,1 bis 10 Gew.%, vorzugsweise 0,1 bis 5 Gew.%, Cholin, Glutamat, Acetat, Dimethylglycin oder Betain als Wachstumssubstrat. Besonders bevorzugt wird Betain zusammen mit Glutamat in Mengen von je 0,2 bis 5 Gew.% eingesetzt.

Des weiteren ist es in der mikrobiologischen Technik üblich, der Vorkultur auch die umzusetzenden Ausgangsverbindungen γ-Butyrobetain, Crotonobetain oder Gemische davon in Mengen von 0,1 bis 10 Gew.%, vorzugsweise 0,1 bis 5 Gew.%, bezogen auf das Reaktionsmedium, zuzusetzen.

Das γ-Butyrobetain bzw. Crotonobetain kann als Hydrochloridsalz, als freies inneres Salz sowie in Form seiner Derivate vorliegen.

Mit den nach oben genannten Verfahren hergestellten Vorkulturen können weitere Kulturen beimpft werden.

Diese weiteren Kulturen haben zweckmässing die gleiche Zusammensetzung wie die Vorkulturen.

Beim erfindungsgemässen Verfahren wird mit dieser Vorkultur ein Bioreaktor angeimpft der ein gleich zusammengesetztes Medium enthält, um die L-Carnitin-Produktion im Batch anzufahren.

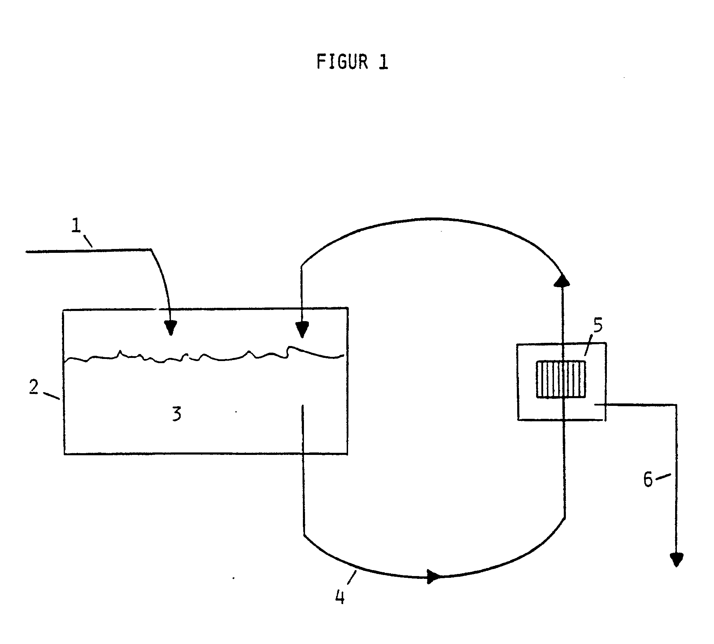

Darauf kann gemäss dem Prinzip nach Figur 1 auf die erfindungsgemässen kontinuierlichen Fahrweise umgestellt werden.

Es wird zweckmässig so vorgegangen, dass mit einer Durchflussrate D von $0,05h^{-1}$ bis $0,5h^{-1}$ vorzugsweise $0,07h^{-1}$ bis $0,12h^{-1}$ gleichzeitig γ-Butyrobetain und / oder Crotonobetain in einer Konzentration von zweckmässig 1 g/l bis 100 g/l vorzugsweise 10 g/l bis 50 g/l und Betain in einer Konzentration von zweckmässig 1 g/l bis 100 g/l vorzugsweise 2 g/l bis 20 g/l durch den Mediumeinlauf 1 in den Bioreaktor 2 gepumpt wird (Konzentrationen bezogen auf 1 l Kulturflüssigkeit).

Die im Reaktor 2 enthaltene Kulturflüssigkeit 3, zweckmässig entsprechend dem vitaminhaltigen Mineralmedium nach [Kulla et. al., Arch. Microbiol. 135, 1 (1983)], enthaltend den L-Carnitin produzierenden Stamm HK 1331b, wird gleichzeitig ausserhalb des Bioreaktors 2 in einem Kreislauf 4, der eine Zellabtrennungseinrichtung 5 (zweckmässig eine Ultrafiltration oder eine Zentrifugation) enthält, geführt.

Durch diese Zellabtrennung 5 wird einerseits erreicht, dass die aktive Biomasse dem Bioreaktor 2 nicht entzogen wird sondern zurückgeführt wird und andererseits, dass dem Kreislauf die L-Carnitin enthaltende zellfreie Lösung 6 entzogen werden kann. Es wird dabei so vorgegangen, dass eine gleich grosse Menge zellfreier Lösung 6 aus der Zellabtrennung abgezogen wird wie im Mediumeinlauf 1 dem Bioreaktor 2 zugeführt wird.

Die Konzentration an L-Carnitin in der zellfreien Lösung 6 ist in der Regel der umgesetzten Menge an γ-Butyrobetain oder Crotonobetain äquivalent.

Die zellfreie Lösung 6 enthält in der Regel noch 5 bis 10% nicht umgesetztes γ-Butyrobetain oder Crotonobetain.

Ueberraschend ist die hohe Stabilität des Systems. So ist über Wochen kein Aktivitätsverlust zu beobachten.

Eine Entlastung der Kultur kann dadurch erreicht werden, dass man das einzusetzende $\gamma$ Butyrobetain und / oder das Crotonobetain vorgängig mittels Ionenaustauscher oder mittels Elektrodialyse entsalzt und reinigt.

Um hundertprozentigen Umsatz zu erreichen kann ebenfalls so vorgegangen werden, dass man eine Nachreaktionsstufe in Form einer Kaskade anordnet.

Die Gewinnung des L-Carnitins aus der zellfreien Lösung 6 kann so durchgeführt werden, dass mittels einer Labor-Elektrodialyseanlage, die Lösung von den geladenen Teilchen (Kationen und Anionen) befreit wird. Der Endpunkt der Entsalzung kann konduktometrisch ermittelt werden. dabei wandern die Salze in den Konzentratkreislauf, während das L-Carnitin als inneres Salz ("Betain") im Diluat-Kreislauf verbleibt. So können Ausbeuten an L-Carnitin im Diluat nach der Entsalzung von mehr als 95% erreicht werden.

Alternativ zur Elektrodialyse kann das L-Carnitin auch mittels eines stark sauren Kationenaustauschers in der $H^+$-Form entsalzt werden (vgl. J.P. Vandecasteele, Appl. Environ. Microbiol. 39, 327 (1980) Dabei lässt man die Lösung solange über eine Ionenaustauscherkolonne fliessen, bis der Ionenaustauscher erschöpft ist und L-Carnitin durchbricht. Die Anionen gehen als freie Säuren in den Durchlauf. Die Kationen bleiben auf dem Ionenaustauscher. Nach neutralwaschen des Ionenaustauschers mit Wasser kann das L-Carnitin mit wässriger Ammoniaklösung eluiert werden. So können Ausbeuten an L-Carnitin im ammoniakalischen Eluat von mehr als 95% erreicht werden.

Die sowohl bei der Elektrodialyse wie auch mittels Ionenaustauscher anfallenden verdünnten L-Carnitin-Lösungen können entweder durch Verdampfung oder auch durch Umkehrosmose aufkonzentriert und anschliessend azeotrop entwässert werden.

Das so anfallende L-Carnitin kann dann durch anschliessende Umkristallisation aus zweckmässig Isobutanol/Aceton, Methanol, Ethanol, n-Butanol bzw. in Kombination mit Lösungsmitteln welche L-Carnitin wenig lösen, wie z. b. Aceton, Ethylacetat, Butylacetat, Isobuthylmethylketon und Acetonitril vorzugsweise Isobutanol und Aktivkohlebehandlung in reines weisses L-Carnitin übergeführt werden. Es kann gemäss diesem Verfahren L-Carnitin mit spezifischen Drehungen von $[\alpha]_D^{25}$ - 30,5 bis - 31,0°, c = 1 in $H_2O$ (Literaturwert -30,9°; Strack et al., Hoppe-Seyler's Z. f. physiolog. Chem., 318 (1960), 129) und einem Gehalt von mehr als 99 % (HPLC) erhalten werden.

Beispiel 1

Eine 0.1 l Vorkultur des Stammes HK 1331b wurde in folgendem Nährmedium bei 30°C, pH7,0 während 24 Stunden kultiviert.

## Zusammensetzung des Nährmediums

| | |
|---|---|
| L-Glutamat | 2 g |
| Betain | 2 g |
| Crotonobetain | 2 g |
| Puffer-Lösung | 100 ml |
| Mg-Ca-Fe-Lösung | 25 ml |
| Spurelementen-Lösung | 1 ml |
| Vitamin-Lösung | 1 ml |
| mit Wasser auf | 1 l |

6

Puffer-Lösung

| | |
|---|---|
| $Na_2SO_4$ | 1 g |
| $Na_2HPO_4.2H_2O$ | 25.08 g |
| $KH_2PO_4$ | 10 g |
| NaCl | 30 g |
| mit Wasser auf | 1 l |

Mg-Ca-Fe-Lösung

| | |
|---|---|
| $MgCl_2.6H_2O$ | 16 g |
| $CaCl_2.2H_2O$ | 0.58 g |
| $FeCl_3.6H_2O$ | 0.032 g |
| mit Wasser auf | 1 l |

Spurelementen-Lösung

| | |
|---|---|
| $ZnSO_4.7H_2O$ | 100 mg |
| $MnCl_2.4H_2O$ | 30 mg |
| $H_3BO_3$ | 300 mg |
| $CoCl_2.6H_2O$ | 200 mg |
| $CuCl_2.2H_2O$ | 10 mg |
| $NiCl_2.6H_2O$ | 22 mg |
| $Na_2MoO_4.2H_2O$ | 30 mg |
| mit Wasser auf | 1 l |

## Vitamin-Lösung

| | |
|---|---|
| Pyridoxal.HCl | 10 mg |
| Riboflavin | 5 mg |
| Nikotinamid | 5 mg |
| Thiamin.HCl | 5 mg |
| Biotin | 2 mg |
| Natriumpantothenat | 5 mg |
| p-Aminobenzoesäure | 5 mg |
| Folsäure | 2 mg |
| Vitamin B 12 | 5 mg |
| mit Wasser auf | 1 l |

Mit der Vorkultur wurde 2 l Nährmedium gleicher Zusammensetzung im Fermentor beimpft und bei 30°C, pH7 während 24 Std. kultiviert. Der pH wurde durch Zugabe von 8%-iger Phosphorsäure bei 7.0 konstant gehalten.

Anschliessend wurde mit dem kontinuierlichem Betrieb begonnen. Mit einer Flussrate D von 0.09/h wurde das oben beschriebene Medium enthaltend 15g/l Betain, 24,3g/l Crotonobetain (mit Hilfe der Elektrodialyse entsalzt), ohne L-Glutamat durch den Mediumeinlauf 1 in den Bioreaktor 2 eingepumpt.

Die Kulturflüssigkeit 3 die das L-Carnitin und die HK 1331b Biomasse enthält, wird kontinuierlich in einem Kreislauf 4, mit einer Geschwindigkeit von 2 l/Min., durch eine übliche Ultrafiltrationseinrichtung durchgepumpt.

Die durch Ultrafiltration getrennte aktive Biomasse wird in den Bioreaktor zurückgeführt.

Das klare L-Carnitin enthaltende Filtrat 6 wurde mit einer Flussrate von ebenfalls 0.09/Std abgepumpt.

Laut Analyse (HPLC) enthielt das Filtrat 25 g/l L-Carnitin und 2.0 g/l nicht umgesetztes Crotonobetain. Dies entspricht einem 92%-igem Umsatz von Crotonobetain und einer L-Carnitin-Ausbeute von 99.6% auf das umgesetzte Crotonobetain bezogen. Das Betain war vollständig katabolisiert.

Nach Erreichen einer maximalen Zelldichte von ca. 35 g Trockengewicht pro Liter blieb die Biomassekonzentration und die L-Carniten-Produktivität während mindestens 1 Monat konstant.

Isolierung von L-Carnitin

Reines L-Carnitin konnte aus der Lösung, welche 25 g L-Carnitin, 2 g Crotonobetain und ca. 10 g anorganische Salze pro Liter Lösung enthielt, wie folgt isoliert werden:

1. Entsalzung

2 lt L-Carnitin-Lösung wurden mittels eines handelsüblichen stark sauren Kationenaustauschers in der H±Form und nachfolgender Elution mit wässrigem Ammoniak entsalzt.

Es wurde wie folgt vorgegangen:

In die Glaskolonne wurden 400g DOWEX® HCR-W2 eingefüllt. Dann liess man 2 lt L-Carnitin-Lösung über den Ionenaustauscher in ca. 3 Std. fliessen. Dabei brach das L-Carnitin noch nicht durch! Danach wurde mit E-Wasser ca. 1,5 lt ausgewaschen. Anschliessend wurde das L-Carnitin und Crotonobetain mit 1 lt 5%-igem Ammoniak und 0,5 lt E-Wasser in ca 3 Std. eluiert, man erhielt 1550ml ammoniakalische entsalzte L-Carnitin-Lösung. Darin wurden 30,9 g L-Carnitin und 2,47 g Crotonobetain pro Liter bestimmt. Dies entspricht einer Ausbeute von 96% bei der Entsalzung mittels Ionenaustauscher.

## 2. Aufkonzentrierung

Die Lösung aus der Entsalzung mit Ionenaustauscher (1550 ml) wurde bei 50°C und 25 mbar mittels eines Labor-Rotationsverdampfers eingeengt. Zur Entfernung des Wassers, wurde dieses azeotrop mit Isobutanol am Rotationsverdampfer unter Vakuum (50°C, 25 mbar) herausgeschleppt und der Rückstand bei 50°C /25 mbar getrocknet (34,05 g) enthaltend 90,7% L-Carnitin und 7,2% Crotonobetain.
Dies entspricht einer praktisch quantitativen Ausbeute an L-Carnitin für die Aufkonzentrierung.

## 3. Reinigung

Die Reinigung des roh L-Carnitins erfolgte auf gleiche Weise wie sie im Beispiel 2 beschrieben ist. Dabei wurden eine vergleichbare Ausbeute und Qualität erreicht.

## Beispiel 2

100 ml des im Beispiel 1 beschriebenen Nährmediums, das anstelle des Crotonobetains 2 g/l Butyrobetain enthielt, wurde mit dem Stamme HK 1331b angeimpft und bei 30°C, pH7 während 24 Std. kultiviert.
Hier wurden im Bioreaktor 2 l des gleichen Nährmediums beimpft und wie die Vorkultur (bei 30°C, pH7) 24 Std lang kultiviert. Darauf wurde mit dem kontinuierlichen Betrieb begonnen. Das Nährmedium, das die folgende Zusammensetzung hatte,

| | |
|---|---|
| Butyrobetain entsalzt | 25 g |
| Betain | 0,7 g |
| $MgCl_2 \cdot 6H_2O$ | 200 mg |
| $CaCl_2 \cdot 2H_2O$ | 14.5 mg |
| $FeCl_3 \cdot 6H_2O$ | 0.8 mg |
| $Na_2SO_4$ | 100 mg |
| KCl | 100 mg |
| Spurelementen-Lösung | 1 ml |
| Vitamin-Lösung | 1 ml |
| mit Wasser auf | 1 l |

wurde mit einer Flussrate von 0.09/Std. in den Bioreaktor eingepumpt. Der pH wurde durch Zugabe von 8%iger $H_3PO_4$ auf 7 konstant gehalten.
Die Kulturflüssigkeit wurde wie im Beispiel 1 beschrieben, kontinuierlich filtriert und das klare Filtrat gesammelt. Nach Erreichen einer maximalen Zellen-Dichte von ca. 30 g Trockengewicht pro Liter blieb die L-Carnitin-Produktivität der Kultur über 4 Wochen konstant. Man erhielt täglich 4.5 l Filtrat. Laut HPLC-Analyse enthielt der Filtrat 25 g/l L-Carnitin und 1.9 g/l Butyrobetain. Dies entspricht einem 92.4%-igem Umsatz des Edukts und einer L-Carnitin Ausbeute von 97.5% auf das umgesetzte Butyrobetain bezogen. Das Betain war vollständig katabolisiert.

## Isolierung von L-Carnitin

Reines L-Carnitin konnte aus der Lösung, welche ca. 25 g L-Carnitin, 2 g Butyrobetain und ca. 4 g anorganische Salze pro Liter Lösung enthielt, wie folgt isoliert werden:

## 1. Entsalzung

2 lt L-Carnitin-Lösung wurden mittels einer üblichen Labor-Elektrodialyseanlage (Berghof BEL-2) entsalzt.

Dabei wurde wie folgt vorgegangen:

In den Elektrodenkreislauf wurden 1 lt 5%-ige Natriumsulfat-Lösung, in den Konzentratkreislauf 1,9 lt 0,1%-ige Kochsalz-Lösung und in den Diluatkreislauf 2,0 lt L-Carnitin eingefüllt. Nun wurde mit einer Spannung welche bei 24 Volt und einem Strom welcher bei 2 A begrenzt war während ca. 4 Std. elektrodialysiert. Umwälzgeschwindigkeit der drei Kreisläufe ca. 1,8 lt/Min. Zu Beginn der Dialyse wurde eine Leitfähigkeit von ca. 5 mS/cm im Dialysatkreislauf (enthalten das L-Carnitin und Butyrobetain) gemessen. Am Ende der Elektrodialyse betrug die Leitfähigkeit ca. 0,1 mS/cm. Die L-Carnitin-Lösung im Diluatkreislauf wurde abgepumpt und mit ca. 200 ml E-Wasser nachgewaschen. In dieser entsalzten L-Carnitin-Lösung (2150 ml) wurden 22,1 g L-Carnitin und 1,77 g Butyrobetain pro Liter analytisch wiedergefunden, was einer Ausbeute von 95% bei der Entsalzung mittels Elektrodialyse entspricht. Diese Lösung wurde zur Aufkonzentrierung verwendet.

## 2. Aufkonzentrierung

Die Lösung aus der Entsalzung mittels Elektrodialyse (10 lt) wurde mittels eines üblichen Umkehrosmose-Moduls von 22 g L-Carnitin auf ca. 160 g L-Carnitin / lt (1 molar) aufkonzentriert.

Dabei wurde wie folgt vorgegangen:

Die zu konzentrierende Lösung wurde in den Arbeitsbehälter eingefüllt und nach Druckaufbau mit Stickstoff auf ca. 42 bar mittels der Umwälzpumpe solange umgewälzt bis ca. 8,61 lt Permeat durchgetreten waren (Zeitbedarf: 2 bis 4 Std.). Im Retentat verblieb eine ca. 7-fach aufkonzentrierte Lösung, enthaltend 152 g L-Carnitin und 12,1 g Butyrobetain. In das Permeat gingen ca. 5 % des L-Carnitins und Butyrobetains, so dass die Ausbeute für die Aufkonzentrierung bei der Umkehrosmose rund 95 % betrug.

Die weitere Aufkonzentrierung der mittels Umkehrosmose angereicherten L-Carnitin-Lösung zu roh L-Carnitin erfolgte analog der unter Beispiel 1 angeführten Methode durch Eindampfung am Rotationsverdampfer und azeotroper Trocknung mit Isobutanol welche praktisch quantitativ verlief.

## 3. Reinigung

60 g roh L-Carnitin (ca. 93 % L-Carnitin und 6 % Butyrobetain) und 6 g Aktivkohle wurden in 900 ml Isobutanol zum Rückfluss erwärmt. Die Aktivkohle wurde heiss abfiltriert und aus dem Filtrat 580 ml Isobutanol abdestilliert, wobei das L-Carnitin teilweise auskristallisierte. Es wurden 300 ml Aceton zugegeben und abgekühlt auf Raumtemperatur. Das L-Carnitin wurde abgenutscht und mit 2 x 60 ml Aceton gewaschen. Die Umkristallisation wurde ein zweites Mal durchgeführt (ohne Kohlebehandlung) um das Butyrobetain vollständig zu entfernen. Danach wurde bei 70° C/25 mbar getrocknet bis zur Gewichtskonstanz. Man isolierte 49.5 g weisses, unverfärbtes L-Carnitin (HPLC mehr als 99 %; spez. Drehung $[\alpha]_D^{25}$ - 30,9°, (c = 1, $H_2O$) in einer Ausbeute von 87 % über beide Umkristallisationen, was einer durchschnittlichen Ausbeute von 93 % pro Umkristallisation entspricht. Die Eindampfrückstände 4,76 g aus der 1. Umkristallisation und 3,46 g aus der 2. Umkristallisation (hauptsächlich L-Carnitin und Butyrobetain) konnten in die Fermentation zurückgeführt werden.

## Patentansprüche

1. Verfahren zur kontinuierlichen Herstellung von L-Carnitin auf mikrobiologischem Weg, dadurch gekennzeichnet, dass man in einem Bioreaktor den Mikroorganismus HK 1331b (DSM Nr. 3225) oder seine Mutanten mit Crotonobetain und/oder γ-Butyrobetain in Gegenwart eines Wachstumssubstrats kultiviert und die Kulturflüssigkeit ausserhalb des Bioreaktors in einem Kreislauf führt, in welchem eine Zellabtrennung durchgeführt wird, wobei dem Bioreaktor eine gleich grosse Menge zellfreier Lösung entzogen wird wie als Substrat dem Bioreaktor zugeführt wird und aus der zellfreien Lösung das L-Carnitin abgetrennt wird.

2. Verfahren nach Patentanspruch 1, dadurch gekennzeichnet, dass Crotonobetain, γ-Butyrobetain oder die Gemische davon in Mengen von 0,1 bis 10 Gew.%, bezogen auf das Kulturmedium, angewendet werden.

3. Verfahren nach Patentansprüchen 1 und 2, dadurch gekennzeichnet, dass als Wachstumssubstrat Dimethylglycin, Cholin, Glutamat, Acetat und/oder Betain verwendet wird.

4. Verfahren nach Patentansprüchen 1 bis 3, dadurch gekennzeichnet, dass die Wachstumssubstrate in Mengen von 0,1 bis 10 Gew.%, bezogen auf das Kulturmedium, angewendet werden.

5. Verfahren nach Patentansprüchen 1 bis 4, dadurch gekennzeichnet, daß die Zellabtrennung durch Zentrifugation oder durch Ultrafiltration erfolgt.

6. Verfahren nach Patentansprüchen 1 bis 5, dadurch gekennzeichnet, daß Durchflußraten von 0.05 - 0.5 $h^{-1}$ angewendet werden.

7. Verfahren nach Ansprüchen 1 bis 6, dadurch gekennzeichnet, daß man ein $\gamma$-Butyrobetain und/oder Crotonobetain einsetzt, das entsalzt und gereinigt ist.

8. Verfahren nach Anspruch 7, dadurch gekennzeichnet, daß das $\gamma$-Butyrobetain und/oder Crotonobetain durch Elektrodialyse oder durch Ionenaustauscher entsalzt und gereinigt ist.

9. Verfahren nach Ansprüchen 1 bis 8, dadurch gekennzeichnet, daß die Isolierung von L-Carnitin aus der zellfreien Lösung durch Kationenaustauscherchromatographie erfolgt.

10. Verfahren nach Ansprüchen 1 bis 8, dadurch gekennzeichnet, daß die Isolierung von L-Carnitin aus der zellfreien Lösung durch Elektrodialyse mit anschließender Umkristallisierung erfolgt.

## Claims

1. Process for the continuous preparation of L-carnitine by microbiological route, characterized in that in a bioreactor microorganism HK 1331b (DSM No. 3225) or its mutants is cultivated with crotonobetaine and/or $\gamma$-butyrobetaine in the presence of a growth substrate and the culture liquid is guided outside the bioreactor in a cycle in which a cell removal is carried out, the same amount of cell-free solution being withdrawm from the bioreactor as is introduced into the bioreactor as substrate, and L-carnitine is separated from the cell-free solution.

2. Process according to patent claim 1, characterized in that crotonobetaine, $\gamma$-butyrobetaine or mixtures thereof are used in amounts of 0.1 to 10 weight-%, based on the culture medium.

3. Process according to patent claims 1 and 2, characterized in that as growth substrate dimethyl glycine, choline, glutamate, acetate and/or betaine is used.

4. Process according to patent claims 1 to 3, characterized in that the growth substrates are used in amounts of 0.1 to 10 weight-%, based on the culture medium.

5. Process according to patent claims 1 to 4, characterized in that the cell removal is carried out by centrifugation or by ultrafiltration.

6. Process according to patent claims 1 to 5, characterized in that flowing through rates of 0.05 to 0.5 $h^{-1}$ are used.

7. Process according to patent claims 1 to 6, characterized in that a $\gamma$-butyrobetaine and/or crotonobetaine is used which has been desalified and purified.

8. Process according to patent claim 7, characterized in that $\gamma$-butyrobetaine and/or crotonobetaine are desalified and purified by electrodialysis or by ion exchange.

9. Process according to patent claims 1 to 8, characterized in that the isolation of L-carnitine from the cell-free solution is carried out by cation exchange chromatography.

10. Process according to patent claims 1 to 8, characterized in that the isolation of L-carnitine from the cell-free solution is carried out by electrodialysis with subsequent recrystallization.

**Revendications**

1. Procédé de préparation en continu de L-carnitine par voie microbiologique, caractérisé en ce que l'on cultive dans un bioréacteur le micro-organisme HK 1331b (DSM N°.3225) ou ses mutants avec la crotonobétaïne et/ou la γ-butyrobétaïne en présence d'un substrat de croissance et l'on introduit le liquide de culture à l'extérieur du bioréacteur dans un circuit dans lequel une séparation des cellules est réalisée, une quantité de solution dépourvue de cellules identique à la quantité de substrat introduite dans le bioréacteur étant prélevée dans le bioréacteur et la L-carnitine étant séparée de la solution dépourvue de cellules.

2. Procédé selon la revendication 1, caractérisé en ce que l'on utilise la crotonobétaïne, la γ-butyrobétaïne ou des mélanges de celles-ci en des quantités de 0,1 à 10% en poids par rapport au milieu de culture.

3. Procédé selon les revendications 1 et 2, caractérisé en ce que l'on utilise comme substrat de culture la diméthylglycine, la choline, un glutamate, un acétate et/ou la bétaïne.

4. Procédé selon les revendications 1 à 3, caractérisé en ce que les substrats de culture sont utilisés en des quantités de 0,1 à 10% en poids par rapport au milieu de culture.

5. Procédé selon les revendications 1 à 4, caractérisé en ce que la séparation des cellules a lieu par centrifugation ou par ultrafiltration.

6. Procédé selon les revendications 1 à 5, caractérisé en ce que l'on utilise des débits d'écoulement de 0,05 à 0,5 $h^{-1}$.

7. Procédé selon les revendications 1 à 6, caractérisé en ce que l'on utilise une γ-butyrobétaïne et/ou une crotonobétaïne qui a subi une élimination des sels et qui est purifiée.

8. Procédé selon la revendication 7, caractérisé en ce que la γ-butyrobétaïne et/ou la crotonobétaïne subit une élimination des sels et est purifiée par électrodialyse ou par échangeur d'ions.

9. Procédé selon les revendications 1 à 8, caractérisé en ce que l'isolement de la L-carnitine à partir de la solution dépourvue de cellules a lieu par chromatographie sur échangeur de cations.

10. Procédé selon les revendications 1 à 8, caractérisé en ce que l'isolement de la L-carnitine à partir de la solution dépourvue de cellules a lieu par électrodialyse puis recristallisation.

FIGUR 1